# EUROPEAN PATENT APPLICATION

(11) **EP 1 046 401 A2**
(43) Date of publication of application: **25.10.2000**
(21) Application number: 00303357.8
(22) Date of filing: 20.04.2000
(51) Int. Cl.: A61K 51/04

(54) **Fatty acid derivative labelled with a radioactive transition metal**

(30) Priority: 20.04.1999 JP 11208699
(71) Applicant: NIHON MEDI-PHYSICS Co., Ltd., Nishinomiya-shi, Hyogo-ken (JP)
(72) Inventor: Saji, Hideo, Kyoto-shi (JP); Magata, Yasuhiro, Takatsuki-shi (JP); Arano, Yasushi, Chiba-shi (JP); Yamamura, Norio, Kawanishi-shi (JP)
(74) Representative: Keen, Celia Mary

(57) **Abstract**

The present invention provides a fatty acid derivative labelled with a radioactive transition metal which is recognised as an energy substrate in a living body and is metabolized by β-oxidation such that any change in said β-oxidation activity can be detected as a change in the elimination rate of the radioactivity from the body. The fatty acid derivative may be of formula (1),

Q-RCOOH (1)

wherein Q is a chelating group capable of forming a neutral complex with a radioactive transition metal, R is a group of the formula -(CR¹R²)ₙ- wherein n is an integer of 4-9; and R¹ and R² are each individually hydrogen or a lower alkyl group, for instance C₁-C₆ alkyl.

## Description

### BACKGROUND OF THE INVENTION

### Field of the invention

The present invention relates to a fatty acid derivative labeled with a radioactive transition metal. More particularly, the invention relates to a fatty acid derivative being labeled with a radioactive transition metal, which is recognized as an energy substrate in living body, and is metabolized by β-oxidation, further the changes of said β-oxidation activity can be detected as the changes of elimination rate of the radioactivity. Thus, the invention relates to a fatty acid derivative which is useful for in vivo radioisotope imaging for diagnosis by detecting the state of energy production in the cells from outside of the body, also the invention relates to a method for preparing the same.

### Related Art

Developments of a compound which can be used for detecting the state of energy production in the cells from outside of the body is quite important for finding the pathological state of living body. In this connection, radioactive transition metals, for example, radioactive technetium (^{99m}Tc) is frequently used as a labeling nuclide for preparing a radiopharmaceutical for the reasons that it posesses a short half-life (6 hr) and emits γ-ray of suitable intensity (141 keV), it is a nuclide obtainable from ⁹⁹Mo/^{99m}Tc generator in low production cost. Introduction of a chelating group into the molecule of mother compound such as fatty acid participating energy production in the cells is necessary for holding a radioactive transition metal element which does not usually exist in living body. However, up to date, a satisfactory compound has not been developed, because there are certain problems in drug design for chelating region, that is, the size, charge and lipophilicity of metal chelate which does not injure the desired property of the mother compound in living body, has not been successfully designed.

An energy for the myocardium cells muscles is produced by oxidation of a fatty acid, for this reason a radiolabeled fatty acid, which efficiently accumulates and is suitably retained in the myocardium, is understood as useful compound for diagnosis of ischemic heart disease and cardiomyopathy. In this connection, compound prepared by attaching an N₂S₂-type bisaminoethanethiol to the ω-position of a long-chain fatty acid having 14 - 16 carbon and coordinated with ^{99m}Tc is now under investigation. However, said compound has not been succeeded to apply for this purpose due to its low accumulation to the heart as compared with the property of [¹²³I]-(p-iodophenylpentadecanoic acid) ([¹²³I]-IPPA) which is now clinically used. Additionally, it has not been confined as yet wheather or not the former compound is actually metabolized by β-oxidation in myocardium. (Mach, R. H., et al: J. Rad. Appl. Instrum. B. 18, 215-226; Jones, G., et al.: Nucl. Med. Biol., 21, 117-123).

It is well known that, in the liver, energy for the parenchymal cell tissues is generally obtained by β-oxidation of a fatty acid, and fatty acid is also esterified. The study using the medium-chain fatty acid labeled with ¹¹C shows that a medium-chain fatty acid derivative is metabolized only by β-oxidation thereof, without esterification, and changes of β-oxidation activity in the liver can be possibly detected as changes of elimination rate of the radioactivity from the outside of living body (Yamamura, et al.: Nucl. Med. Biol., 25, 467-472). However, there have not been known as yet whether or not a fatty acid derivative labeled with a radioactive transition metal which is bonded to a chelating group is metabolized by β-oxidation only.

### SUMMARY OF THE INVENTION

In view of these circumstances, an object of the present invention is to provide a fatty acid derivative, being labeled with a radioactive transition metal, which is recognized as an energy substrate in living body, and is metabolized by β-oxidation so that the state of energy metabolism thereof can be detected from outside of the body.

Concretely, said fatty acid derivative being labled with a radoactive transition metal is a compound having a straight-chain or branched-chain and represented by the following general formula (1),

Q-RCOOH (1)

wherein, Q is a chelating group forming a neutral complex bonded with a radioactive transition element; R is a group of the formula R=-(CR¹R²)ₙ-, wherein n is 4-9; and R¹ and R² are each individually a hydrogen atom or a lower alkyl group.

Q is selected from the group consisting of bisaminoethanethiol, monoamino-monoamido-bisthiol or bisamido-bisthiol. Preferably, N-[[[2-[(triphenylmethyl)thio]ethyl]amino]acetyl]-S-(triphenylmethyl)-2-aminoethanethiol is exemplified. The radioactive transition metal is selected from technetium or rhenium, preferably technetium is used.

Other embodiment of the present invention is methods for preparing fatty acid derivatives having a straight-chain or branched-chain alkyl group, and represented by the following formula (1),

Q-RCOOH (1)

wherein Q is a chelating group forming a neutral complex bonded with a radioactive transition element; R is a group of the formula R=-(CR¹R²)ₙ-, wherein n is 4-9; and R¹ and R² are each individually a hydrogen atom or a lower alkyl group, which comprises a step of reacting cysteamine with a halogenated acetyl halide to synthesize a mono-aminomonoamide compound, and a step of reacting said monoaminomonoamide compound with a ω-halogenated fatty acid ester. The halogen is selected from any one of chlorine, bromine, iodine and fluorine, and bromine is preferable.

According to the present invention, it can be provided that a fatty acid derivative being labeled with a radioactive transition metal, which is recognized as an energy substrate in living body, and is subjected to β-oxidation and is metabolized, further the changes of said β-oxidation activity can be detected, from outside of the body, as changes of elimination rate of the radioactivity.

### BRIEF DESCRIPTION OF THE DRAWINGS

Fig. 1 shows scintigrams of SPECT (single photon emission computed tomography) in rats.
Fig. 2 shows chromatograms of radioactive metabolites in the urine samples by HPLC (high performance liquid phase chromatography).
Fig. 3 shows chromatograms of in vitro metabolites obtained from the liver slice samples by HPLC.
Fig. 4 shows chromatogram of [^{99m}Tc]MAMA-BA and [^{99m}Tc]MAG-HA by reversed phase HPLC.

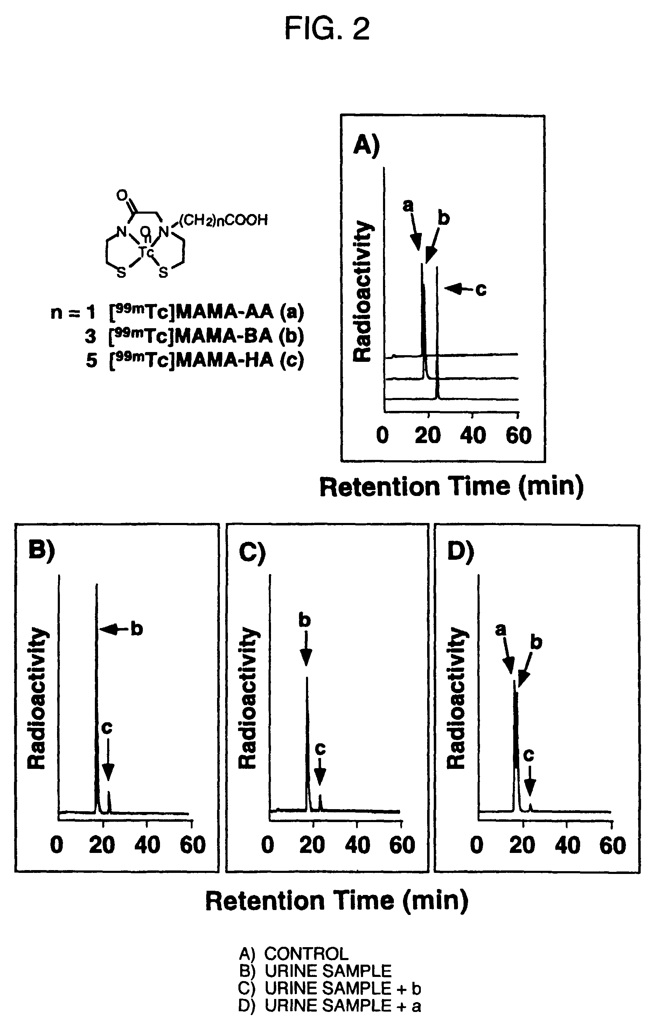

In the Figs. 2 to 4, (a) shows [^{99m}Tc]MAMA-AA, (b) shows [^{99m}Tc]MAMA-BA, (c) shows [^{99m}Tc]MAMA-HA and (d) shows [^{99m}Tc]MAG-HA.

### DETAILED DESCRIPTION OF THE INVENTION

The present invention relates to a fatty acid derivative being labeled with a radioactive transition metal, which is recognized as an energy substrate in living body, and is metabolized by β-oxidation, so that the changes of β-oxidation activity can be detected as changes of the elimination rate of radioactivity, from outside of the body.

There have been known that a medium-chain fatty acid derivative being labeled with ¹¹C or ¹²³I is metabolized by β-oxidation in the liver without esterification, and changes of said β-oxidation activity can be possibly detected, from outside of the body, as changes of elimination rate of radioactivity. However, it has not been known yet that the fatty acid labeled with transition metal is metabolized by the pathway decribed above. In order to labeling a fatty acid with a radioactive transition metal, it is necessary to combine a fatty acid with a chelating group being capable of forming a stable and small mononuclear complex with a radioactive transition metal.

In the present invention, a fatty acid derivative being labled with a radioactive transition metal is a compound having a straight-chain or branched-chain alkyl group and represented by the following general formula (1),

Q-RCOOH (1)

wherein, Q is a chelating group forming a neutral complex bonded with a radioactive transition element; R is a group of the formula R=-(CR¹R²)ₙ-, wherein n is 4-9; and R¹ and R² are each individually a hydrogen atom or a lower alkyl group.

Chelating group of Q forms a neutral and stable mononuclear complex with a radioactive transition metal, further said chelating group may preferably have relatively higher lipophilicity. For example, N₂S₂-type of bisaminoethanethiol, mono-aminemonoamide, bisamidobisthiol and the like may be mentioned. Concretely, N-(2-mercaptoethyl)-N'-methyl-2'-mercaptopropylethylenediamine, N-(2''-methyl-2''-mercaptopropyl)-2-[2'-methyl-2'-(mercaptopropyl)-ethylenediamine, N,N'-bis(2-mercaptoethyl)ethylenediamine, N-(2''-methyl-2''-mercaptomethylpropyl)-2-[(2'-mercaptoethyl)amino]acetamide, N-(2''-mercaptoethyl)-2-[(2'-mercaptoethyl)amino]acetamide, N-(2''-methyl-2''-mercaptopropyl)-2-[(2'-methyl-2'-mercaptopropyl)amino]-acetamide, N,N'-bis(mercaptoacetyl)ethylenediamine and the like can be exemplified.

The fatty acid is a fatty acid having a straight-chain or a branched-chain, and preferably pentanoic acid, hexanoic acid, heptanoic acid, octanoic acid, nonanoic acid, decanoic acid, 3-methylbutanoic acid, 2-dimethylpropanoic acid, 2-methyloctanoic acid, 3-dimethyloctanoic acid and the like can be exemplified. Attached position of the fatty acid to the chelating group may be any one of the carbon atom or nitrogen atom which is the constitutional element of chelating group. As to the radioactive transition metal to form complex with the chelating group of Q, technetium or rhenium may be used, particularly technetium is preferable.

In the present invention, as to one of the embodiments of method for preparing a fatty acid derivative being bonded to chelating group which forms neutral and stable small mononuclear complex with radioactive transition metal, there can be mentioned a method for preparing a fatty acid derivative having a straight-chain or branched chain alkyl group represented by the general formula (1),

Q-RCOOH (1)

wherein Q is a chelating group forming a neutral complex bonded with a radioactive transition element; R is a group of the formula R=-(CR¹R²)ₙ-, wherein n is 4-9; and R¹ and R² are each individually a hydrogen atom or a lower alkyl group, which comprises a step of reacting cysteamine with a halogenated acetyl halide to synthesize a monoaminemonoamide compound, and a step of reacting said monoaminemonoamide compound with a ω-halogenated fatty acid ester. The halogen is selected from any one of chlorine, bromine, iodine and fluorine, and bromine is preferable.

For example, as shown in the following scheme (2), a compound being coordinated with ^{99m}Tc ([^{99m}Tc]MAMA-HA) is synthesized by reacting hexanoic acid with N-(2''-mercaptoethyl)-2-[(2'-mercaptoethyl)amino]acetamide (MAMA).

First, cysteamine hydrochloride (1) is reacted with trityl chloride so as to protect the thiol group of cysteamine by trityl group (Tr), then the reaction product is dissolved in chloroform, and triethylamine is added thereto. This solution is added dropwise under stirring to a chloroform solution of bromoacetyl bromide which is cooled at -78°C. There is obtained N-[[[2-[(triphenylmethyl)thio]ethyl]amino]acetyl]-S-(triphenylmethyl)-2-aminoethanethiol (Tr-MAMA (3)).

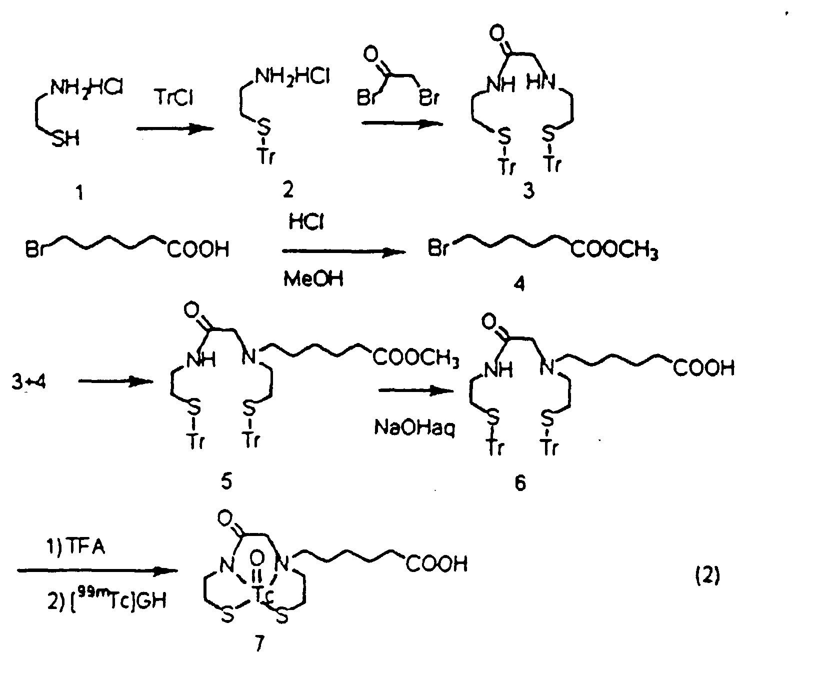

Next, the obtaind compound of Tr-MAMA (3) and diisopropylethylamine are dissolved in acetonitrile, then an acetonitrile solution (8mL) of methyl 6-bromohexenate is added dropwise thereto under stirring. The obtained N-[[[[2-[(triphenylmethyl)thio]ethyl]-amino]carbonyl]methyl]-N-[2-[(triphenylmethyl)thio]-ethyl]amino-6-hexanoic acid methyl ester (5) is hydrolyzed with 5% sodium hydroxide aqueous solution, there can be obtained quantitatively N-[[[[2-[(triphenylmethyl)thio]ethyl]amino]carbonyl]methyl]-N-[2-[(triphenylmethyl)thio]ethyl]amino-6-hexanoic acid (Tr-MAMA-HA (6)). The thus obtained Tr-MAMA-HA (6) is subjected to detritylation by use of TFA containing triethylsilane, then exchange the ligand of [^{99m}Tc] glucoheptanate to obtain [^{99m}Tc]MAMA-HA (7).

Similarly, in order to investigate metabolism of [^{99m}Tc]MAMA-HA, there is synthesized N-[[[[2-[(triphenylmethyl)thio]ethyl]amino]carbonyl]methyl]-N-[2-[(triphenylmethyl)thio]ethyl]amino-4-butyric acid (Tr-MAMA-BA) and N-[[[[2-[(triphenylmethyl)thio]ethyl]-amino]carbonyl]methyl]-N-(2-[(triphenylmethyl)thio]-ethyl]amino-4-acetic acid (Tr-MAMA-AA), then each of them are labeled with ^{99m}Tc. The resulting compounds are used as the authentic samples of these metabolites.

On the other hand, similar to the case of MAMA, as shown in the following scheme (3), a water soluble fatty acid derivative ([^{99m}Tc]MAG-HA) being labeled with ^{99m}Tc is synthesized by bonding hexanoic acid with N₃S-type mercaptoacetylglycylglycylglycine (MAG) which forms a stable and small complex with ^{99m}Tc, said complex having monovalent negative charge. Thus, S-acetylmercaptoacetic acid (9), which is synthesized from mercaptoacetic acid, and N-hydroxysuccinimide (NHS) are dissolved in terahydrofuran (THF), and the solution of the mixture is stirred for 10 minutes at room temperature. After ice-cooled, to this reaction mixture was added a THF solution of dicyclohexylcarbodiimide (DCC), the reaction mixture is stirred for overnight at room temperature. The formed DCC-urea is removed by filtration, the solvent is removed by distillation from the filtrate and dried under reduced pressure. The residue is washed with hexane in several times. There is obtained N-hydroxysuccinimide ester of S-acetylthioglycolic acid (SATA) (10) as in the form of white crystals.

Next, the amino group of glycylglycine is protected with Boc group, a compound (11) is synthesized in which the carboxyl group is converted into N-succinimide ester in the presence of DCC. The obtained compound (11) is dissolved in acetonitrile, and to 0.2M sodium hydroxide aqueous solution in which 6-aminohexanoic acid is dissolved this solution is added dropwise at 50-60°C, followed by reflux for 2 hours. Then the reaction mixture is cooled to 0°C, and the pH is adjusted to 3, and extracted with ethyl acetate. The organic layer is dried over anhydrous calcium sulfate, the solvent is removed by distillation under reduced pressure, then the residue is treated with trifluoroacetic acid (TFA) containing anisol to remove Boc group. TFA is removed by distillation under reduced pressure, the neutralized solution is added dropwise to a DMF solution of SATA, there is obtained S-acetylmercaptoacetylglycylglycylaminohexanoic acid (S-acetyl MAG-HA) (13). Then the ligand of [^{99m}Tc] glucoheptanate is exchanged to S-acetyl MAG-HA to obtain [^{99m}Tc]MAG-HA (14).

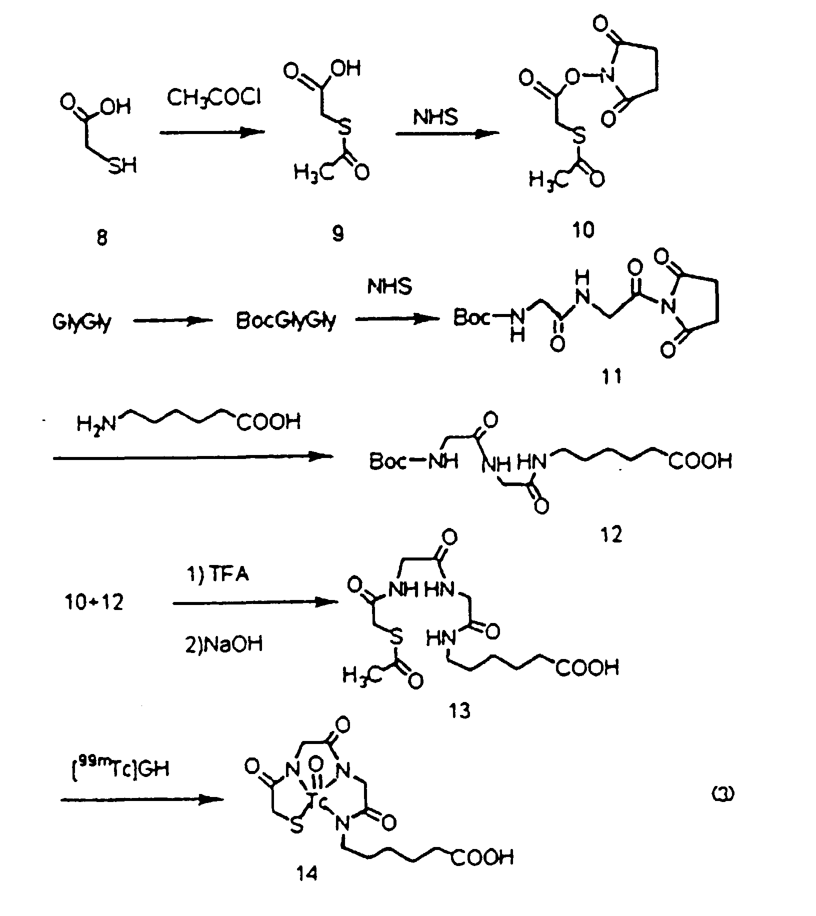

As the result of biodistribution of MAMA-HA labeled with ^{99m}Tc in normal rats, after administration of [^{99m}Tc]MAMA-HA, rapid accumulation followed by rapid elimination of the radioactivity in the liver is observed. Further, the radioactivity was also quickly eliminated from the blood, and the radioactivity washed out from the liver was tranferred to the kidney then to the bladder, and the radioactive metabolite showed the property of being efficiently excreted into urine. While, remarkable accumulation of the radioactivities were not observed in other organs including intestine.

As the results of reversed phase HPLC analysis of the radioactive metabolites in urine sample obtained 60 minutes after the administration of [^{99m}Tc]MAMA-HA, it was clearly observed that the most portion of the radioactivity in urine was eluted at the same retention time of [^{99m}Tc]MAMA-HA. This fact was confirmed by simultaneous analysis conducted by use of the authentic samples of [^{99m}Tc]MAMA-BA or [^{99m}Tc]MAMA-AA, both of them and the urine samples were prepared separately. Additionaly, [^{99m}Tc]MAMA-BA administered in rats hardly accumulated in the liver, and was excreted directly from the kidney to the bladder, therefore, redistribution of [^{99m}Tc]MAMA-BA formed by metabolism of [^{99m}Tc]MAMA-HA to the liver can be negligible. This fact suggests that [^{99m}Tc]MAMA-HA administered to the living body is metabolized in the liver via β-oxidation to [^{99m}Tc]MAMA-BA which is excreted into urine.

The fact that [^{99m}Tc]MAMA-HA is metabolized to [^{99m}Tc]MAMA-BA by β-oxidation was confirmed by an in vitro metabolism test using a piece of liver slice. When a pieace of liver slice and [^{99m}Tc]MAMA-HA were incubated in a buffer solution, then [^{99m}Tc]MAMA-BA was detected as the only radioactive metabolite. On the other hand, when another incubation was conducted similarly, except by adding 2-bromooctanoic acid as an inhibitor for the β-oxidation, then the formation of [^{99m}Tc]MAMA-BA was inhibited completely. The result of this test suggests that [^{99m}Tc]MAMA-HA was recognized by the enzyme relating to β-oxidation in the liver and was metabolized to form [^{99m}Tc]MAMA-BA.

The final radioactive metabolite of [^{99m}Tc]-MAMA-HA formed by the β-oxidation was confirmed as [^{99m}Tc]MAMA-BA but not [^{99m}Tc]MAMA-AA by means of a reversed phase HPLC conducted simultaneous analysis of the urinary metabolites and the authentic sample. This fact can be understood that, by introducing MAMA to hexanoic acid, recognition of the enzyme relating to the β-oxidation of [^{99m}Tc]MAMA-BA, which is one of the metabolites of [^{99m}Tc]MAMA-HA, was inhibited and oxidation of [^{99m}Tc]MAMA-HA was not proceeded up to [^{99m}Tc]MAMA-AA, thus [^{99m}Tc]MAMA-BA was formed as the final metabolite of [^{99m}Tc]MAMA-HA. As explained above, it was clearly confirmed that surprizingly, the final radioactive metabolite formed by the β-oxidation of [^{99m}Tc]MAMA-HA having the side-chain of 6 carbon atoms was not [^{99m}Tc]MAMA-AA having the side-chain of 2 carbon atoms, but was [^{99m}Tc]MAMA-BA having the side-chain of 4 carbon atoms.

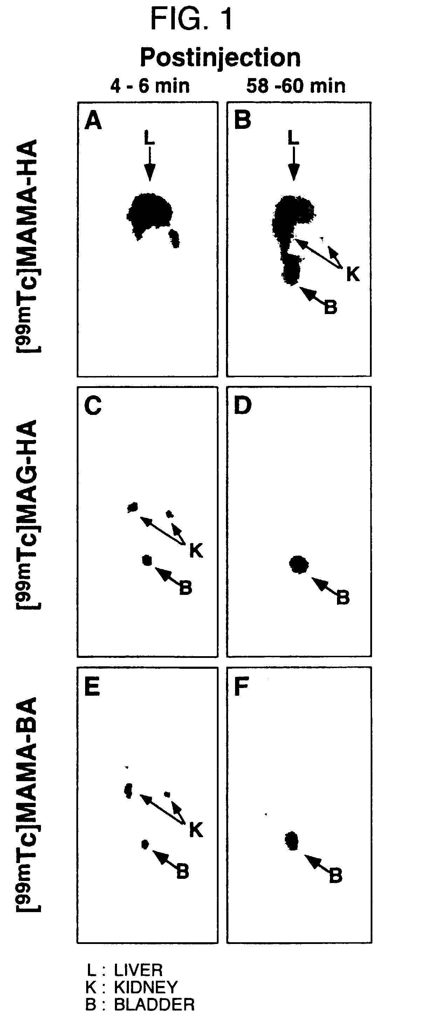

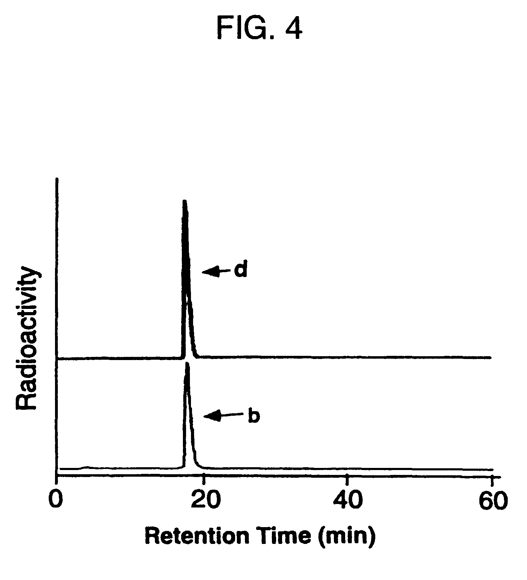

On the other hand, after the administration, [^{99m}Tc]MAG-HA hardly accumulated in the liver (Fig. 1, C and D). As shown in Table 1, partition coefficient (octanol/water) of [^{99m}Tc]MAG-HA and [^{99m}Tc]MAMA-HA indicates that water-solubility of [^{99m}Tc]MAG-HA is higher than that of [^{99m}Tc]MAMA-HA. In case of reversed phase HPLC analysis conducted under the same conditions, [^{99m}Tc]MAG-HA was eluted within almost the same retention time as in the case of [^{99m}Tc]MAMA-BA (Fig. 4). Further, [^{99m}Tc]MAMA-BA hardly accumulated in the liver after the administration in rat. Thus it was understood that the charge of the chelating region of [^{99m}Tc]MAG may be one of the important factors, but the higher water-solubility of [^{99m}Tc]MAG-HA may be the more important factor of lower accumulation in the liver.

In accordance with the present invention, it becomes apparent that, after administration in vivo, [^{99m}Tc]MAMA-HA was taken up by the liver and was metabolized to [^{99m}Tc]MAMA-BA by the β-oxidation, but was not metabolized up to [^{99m}Tc]MAMA-AA. In becomes also apparent that [^{99m}Tc]MAMA-HA is the first fatty acid labeled with radioactive transition metal, which is recognized and metabolized as energy substrate in the living body. Furthermore, suitable selection of the lipophilicity of the chelating group introduced to this compound enables accumulation of this compound to specific organ and rapid urinary excretion of resulting radiometabolite. Thus, by introducing a chelating group labeled with a suitable radioactive transition metal to the fatty acid, the fatty acid labeled with radioactive transition metal is recognized as energy substrate in the living body, and changes of elimination rate of the radiactivity can be detected as changes of activity of the tissue from outside of the body. The method for synthesizing MAMA-HA according to the present invention can be applied easily to synthesis of other fatty acid derivatives having different carbon chains, thus, the present invention is useful and effective to development of various fatty acids being labeled with radioactive transition metals used as radiopharmaceutical for medicinal diagnostic imagings.

### EXAMPLES

The present invention will be explained in more detail by illustrating the following examples, but the invention will not be restricted only to these examples.

Methods for testing the compounds prepared and reagents used are as follows.
(1) ¹H-NMR: JMN-EX270 (manufactured by JEOL Co.), chemical shift values were determined by referring to tetramethylsilane as the internal standard.
(2) Mass spectrum: Determined by use of JMS-HX110A model (manufactured by JEOL Co.).
(3) Reversed phase HPLC: Reversed phase column "Cosmosil 5C18-AR-300 (150 mm X 4.6mm, manufactured by Nacalai Tesque, Inc.) was used.
(4) TLC: Silica gel plate (Merck Art 5553; manufacture by Merck & Co.) was used.
(5) ^{99m}TcO₄-: Prepared by use of ^{99m}Mo/^{99m}Tc generator (Ultra Technecow; manufacture by Daiichi Radioisotope Research Laboratry), and used its eluate as in the form of a physiological saline solution.
(6) Reagents: The all reagents used were Extra-pure reagent grade.
(7) Test animals: The all test animals used were Wister-strain male rats (body weight: 200-250 g). Prior to the test, animals were breeded under light-dark cyclic condition in every 12 hours for 1 week, and had free access to food and water.

### Example 1

### Synthesis of N-[[[2-[(triphenylmethyl)thio]ethyl]- amino]acetyl]-S-(triphenylmethyl)-2-aminoethanethiol (Tr-MAMA) (3)

Cysteamine hydrochloride (11.1 g, 97.7 mmol) was stirred with 150 mL of N,N-dimethylformamide (DMF) containing trityl chloride (27.3 g, 97.9 mmol) at room temperature for 48 hours, so that the thiol group of cysteamine was protected with trityl group. After the reaction, the solvent was removed by distillation under reduced pressure, the resulting residue was re-dissolved in ethyl acetate, under ice-cooling, by adding an aqueous solution saturated with sodium hydrogen carbonate, there was obtained S-tritylcysteamine as white crystals. The obtained crystals were well dried, then 9.97 g (28 mmol) of this compound was dissolved in 50 mL of chloroform, and added triethylamine (14 mL) and stirred. The resulting solution was added dropwise with stirring to a chloroform solution (40 mL) of bromoacetyl bromide (2.84 g, 14 mmol) which was cooled to -78°C. The reaction mixture was stirred at -78°C for 30 minutes, then at room temperature for 24 hours. The reaction mixture was washed with a diluted sulfuric acid of pH 3, an aqueous solution saturated with sodium hydrogen carbonate and an aqueous solution of sodium chloride in this order, after drying the organic layer over anhydrous calcium sulfate, and concentrated under reduced pressure. The residue was roughly purified by a silica gel chromatography using chloroform as an eluting solvent, further, subjected to a silica gel chromatography using ethyl acetate:hexane (95:5) as an eluting solvent, there was obtained 0.95 g of compound of Tr-MAMA (3) as in the form of pale yellow oily product. Yield: 10%.
- ¹H-NMR (CDCl₃):: δ7.44-7.15 (overlapped m, 30H), 3.07 (td, 2H), 3.02 (s, 2H), 2.45 (t, 2H), 2.45 (t, 2H), 2.38-2.31 (overlapped m, 4H).
- FABMS:: Calculated: m/z 679, Found: 679

### Example 2

### Synthesis of N-[[[[2-[(triphenylmethyl)thio]ethyl]-amino]carbonyl]methyl]-N-[2[(triphenylmethyl)thio]-ethyl]amino-6-hexanoic acid methyl ester (5)

An acetonitrile solution (8 mL) of methyl 6-bromohexanate (4) (160 mg, 0.77 mmol) prepared by stirring 6-bromohexanoic acid in methanol containing hydrogen chloride was added dropwise with stirring to a solution prepared by dissolving compound Tr-MAMA (400 mg, 0.59 mmol) and diisopropylethylamine (120 mg, 1.84 mmol) in 2 mL of acetonitrile. 48 Hours after stirring at room temperature, the solvent was removed by distillation under reduced pressure, then the residue was subjected to a silica gel chromatography using ethyl acetate:hexane (95:5) as an eluting solvent, there was obtained 117 mg of compound (5) as in the form of pale yellow oily product. Yield: 24.6%.
- ¹H-NMR (CDCl₃):: δ7.43-7.15 (overlapped m, 30H), 3.64 (s, 3H), 3.01 (td, 2H), 2.82 (s, 2H), 2.40-2.33 (overlapped m, 4H), 2.28-2.22 (overlapped m, 6H), 1.59-1.48 (m, 2H), 1.39-1.19 (overlapped m, 4H).
- FABMS:: Calculated: C₅₁H₅₄N₂O₃S₂(M+H⁺):m/z 807,
Found: 807

### Example 3

### Synthesis of N-[[[[2-[(triphenylmethyl)thio]ethyl]- amino]carbonyl]methyl]-N-[2-[(triphenylmethyl)thio]-ethyl]amino-6-hexanoic acid (Tr-MAMA-HA) (6)

By hydrolyzing compound (5) prepared in Example 2 with an aqueous solution of 5% sodium hydroxide, Tr-MAMA-HA (6) was quantitatively obtained.
- ¹H-NMR (CDCl₃):: δ7.40-7.14 (overlapped m, 30H), 2.99 (td, 2H), 2.87 (s, 2H), 2.40-2.33 (overlapped m, 4H), 2.28-2.20 (overlapped m, 6H), 1.59-1.48 (m, 2H), 1.32-1.21 (overlapped m, 4H).
- FABMS:: Calculated: C₅₀H₅₂N₂O₃S₂(M+H⁺):m/z 793,
Found: 793

### Example 4

### Synthesis of N-[[[[2-[(triphenylmethyl)thio]ethyl]-amino]carbonyl]methyl]-N-[2-[(triphenylmethyl)thiol-ethyl]amino-4-butyric acid (Tr-MAMA-BA)

According to the method similar to that of Example 3 for the compound (6), this compound was synthesized by using Tr-MAMA as the raw material. Tr-MAMA was reacted by adding ethyl 4-bromobutyrate, then the reaction product was purified by a silica gel chromatography using ethyl acetate:hexane (95:5) as an eluting solvent, and hydrolyzed the methyl ester purified by an aqueous solution of 5% sodium hydroxide, Tr-MAMA-BA was obtained in the form of yellow oily product. Yeild was 3.3%.
- ¹H-NMR (CDCl₃):: δ7.40-7.17 (overlapped m, 30H), 2.99 (td, 2H), 2.87 (s, 2H), 2.36-2.33 (overlapped m, 4H), 2.30-2.23 (overlapped m, 6H), 1.65-1.62 (m, 2H),
- FABMS:: Calculated: C₄₈H₄₈N₂O₃S₂(M+H⁺) : m/z 787,
Found: 787

### Example 5

### Synthesis of N-[[[[2-[(triphenylmethyl)thio]ethyl]-amino]carbonyl]methyl]-N-[2-[(triphenylmethyl)thio]-ethyl]amino-4-acetic acid (Tr-MAMA-AA)

According to the method similar to that of Example 3 for the compound (6), this compound was synthesized by using Tr-MAMA as the raw material. Thus, Tr-MAMA was reacted by adding methyl 2-bromoacetate, then the reaction product was purified by a silica gel chromatography using ethyl acetate:hexane (95:5) as an eluting solvent, further hydrolyzed the methyl ester by an aqueous solution of 5% sodium hydroxide, Tr-MAMA-AA was obtained in the form of yellow oily product. Yeild was 12.7%.
- ¹H-NMR (CDCl₃):: δ7.39-7.14 (overlapped m, 30H), 3.10 (s, 2H), 2.99 (s, 2H), 2.98 (td, 2H), 2.50 (t, 2H), 2.34 (t, 2H), 2.24 (t, 2H)
- FABMS:: Calculated: C₄₆H₄₄N₂O₃S₂(M+H⁺):m/z 759,
Found: 759

### Example 6

### Synthesis of Acetylthioglycolic acid N-hydroxysuccinimide ester (SATA) (10)

According to the method of Benary (Benary, E.: Thiotetronic acid and derivatives. Ber., 46, 2103-2107), S-acetylmercaptoacetic acid was synthesized from mercaptoacetic acid.

S-acetylmercaptoacetic acid (9) (5.15 g, 38.15 mmol) and N-hydroxysuccinimide (NHS) (4.45 g, 38.5 mmol) were dissolved in 50 mL of tetrahydrofuran (THF), this mixture solution was stirred at room temperature for 10 minutes. After ice-cooling, to this reaction mixture was added a solution prepared by dissolving dicyclohexylcarbodiimide (DCC) (7.95 g, 38.5 mmol) in 5 mL of THF, and stirred overnight at room temperature. DCC-urea formed was removed by filtration, and the filtrate was dried after evaporating the solvent under reduced pressure. The residue was washed with hexane in several times, 7.69 g of SATA was obtained in the form of white crystals. Yield was 86.3%.
- ¹H-NMR (CDCl₃):: δ3.99 (s, 2H), 2.85 (s, 4H), 2.43 (s, 3H).

### Example 7

### Synthesis of S-acetylmercaptoglycylglycylamino hexanoic acid (S-acetyl MAG-HA) (13)

Compound (11), in which the amino group of glycylglycine was protected with Boc group and the carboxyl group was esterified with N-succinimide in the presence of DCC, was synthesized. Compound (11) obtained was dissolved in 30 mL of acetonitrile, this solution was added dropwise to 0.2 M sodium hydroxide aqueous solution containing 6-aminohexanoic acid (0.79 mg, 38.5 mmol) followed by refluxing for 2 hours. After cooled the reaction mixture to 0°C, the pH of the mixture was adjusted to 3 by a diluted sulfuric acid, then was extracted with ethyl acetate. The organic layer was dried over anhydrous calcium sulfate, the solvent was removed by evaporation under reduced pressure, the Boc group was removed by treating 1.41 g of compound (12) with trifluoroacetic acid (TFA) containing 5% of anisol. After removal of TFA by distillation under reduced pressure, the residue was neutralized with an aqueous solution prepared by dissolving sodium hydroxide in 20 mL of distilled water. This solution was added dropwise to a solution prepared by dissolving SATA (1.04 g, 4.5 mmol) in 20 mL of DMF, then was stirred at room temperature for 2 hours. After removal of the solvent, 396 mg of S-acetyl MAG-HA (13) was obtained in the form of white crystals. Yield was 29.1%.
- ¹H-NMR (CDCl₃):: δ8.42 (t, 1H), 8.13 (t, 1H) 7.70 (t, 1H), 3.72 (d, 2H), 3.66 (s, 2H), 3.65 (d, 2H), 3.03 (td, 2H), 2.36 (s, 3H), 2.19 (t, 2H), 1.54-1.21 (overlapped m, 6H)
- FABMS:: Calculated: C₁₄H₂₃N₃O₆S(M+H⁺): m/z 362,
Found: 362

### Example 8

### Labeling with ^{99m}Tc

Formation of complex of each one of the ligands obtained respectively in Examples 1-7 with ^{99m}Tc was carried out by ligand exchanging reaction with [^{99m}Tc] glucoheptanate ([^{99m}Tc]GH). Previously prepared lyophylized powder of Sn (II) GH (prepared by dissolving 4 g of glucoheptanate (GH) and 1.2 mg of SnCl₂ in 50mL of phosphate buffer of pH 8.6) was dissolved in physiological saline solution of ^{99m}TcO₄⁻ eluted from ⁹⁹Mo/^{99m}Tc generator. The final concentration of GH was 3-5 mg/mL. The solution was allowed to stand at room temperature for 10 minutes, the formation of [^{99m}Tc]GH was confirmed by a TLC method using acetone as a developing solvent. Rf=0.0:[^{99m}Tc]GH, Rf=1.0:^{99m}TcO₄⁻.

In a reaction vial, Tr-MAMA-HA (1 mg) was detritylated by treating with TFA containing 5% of triethylsilane. After removal of TFA, this solution was neutralized with 1M sodium hydroxide aqueous solution, further 0.4 mL of phosphate buffer (0.1 M, pH 8.0) was added thereto. To this solution, the above-mentioned [^{99m}Tc]GH solution (0.4 mL) was added and well stirred, and it was allowed to stand at room temperature for 1 hour. [^{99m}Tc]MAMA-HA was purified by reversed phase HPLC, under the conditions of flow rate 1 mL/min., column temperature: 30°C, employing linear gradient technique starting from a mixture of 0.05 M phosphate buffer solution (pH 7.0) and methanol (90:10) to 100% methanol as a mobile phase within 60 minutes (retention time: 23 minutes).

Similarly, [^{99m}Tc]MAMA-BA and [^{99m}Tc]MAMA-AA were obtained (retention time [^{99m}Tc]MAMA-BA: 17 minutes, and [^{99m}Tc]MAMA-AA: 16 minutes).

Labeling of MAG-HA with ^{99m}Tc was carried out as follows, 3 mg of S-acetyl protected product (13) was dissolved in 1 mL of 0.1 M phosphate buffer (pH 8.0) followed by adding under stirring the same quantity of saline solution of [^{99m}Tc]GH, then this reaction mixture was heated at 90-95°C for 30 minutes. After cooling, [^{99m}Tc]MAG-HA was purified by reversed phase HPLC, under the conditions of flow rate 1 mL/min., column temperature: 30°C, employing linear gradient technique starting from a mixture of 0.05 M phosphate buffer (pH 7.0) and methanol (90:10) to 50% methanol within 30 minutes as mobile phase (retention time: 18 minutes).

Radiochemical yield and radiochemical purity of each one of these fatty acid derivatives labeled with ^{99m}Tc were determined by reversed phase HPLC employed for their purification as well as TLC using acetone as a developing solvent. (Rf=0.75:[^{99m}Tc]MAMA-HA, Rf=0.58:[^{99m}Tc]MAG).

### Example 9

### Determination of partition coefficients

Partition coefficients of [^{99m}Tc]MAMA-HA and [^{99m}Tc]MAG-HA were determined as follows.

Into each one of test tubes containing 3 g each of 1-octanol and 0.1 M-phosphate buffer (pH 7.4), 10 µℓ of a solution containing [^{99m}Tc]MAMA-HA or [^{99m}Tc]MAG-HA was added. The test tube was subjected to the following operations repeated in 3 times that by shaken for 1 minute in 3 times by "VORTEX MIXER" followed by being allowed to stand at room temperature for 20 minutes. Then the each test tube was centrifuged at 1000 × g for 5 minutes. One ml of the sample was taken from each one of the organic layer and aqueous layer. Radioactivities of the samples were determined respectively. Partition coefficients are shown as cpm/g of 1-octanol/buffer solution. Lipophilicity of [^{99m}Tc]MAMA-HA was slightly higher than that of [^{99m}Tc]MAG-HA, but the difference was regarded as significant. The results are shown in Table 1.

**Table 1**

| Partition coefficients of fatty acid derivatives labeled with [^{99m}Tc] | |
|---|---|
| | Partition coefficients(PC) |
| [^{99m}Tc]MAMA-HA | 1.06 ± 0.10 |
| [^{99m}Tc]MAG-HA | 0.89 ± 0.03* |
| Partition coefficient: Octanol/phosphate buffer (pH 7.4) | |

| | |
|---|---|
| * P<0.05 (comparing with [^{99m}Tc]MAMA-HA) | |

### Example 10

### Imaging study in rats by SPECT

Biodistribution of a fatty acid derivative labeled with ^{99m}Tc in rat was imaged by SPECT (single photon emission computed tomography).

After anesthetized with pentobarbital, each one of a fatty acid derivative labeled with ^{99m}Tc (15 MBq/0.5 mL physiolocal saline) was administered respectively to the caudal vein of rat. From this point, dynamic data acquisition (20 seconds X 30 frames, 1 minute X 10 frames and 2 minutes X 20 frames) was initiated.

After the administration, [^{99m}Tc]MAMA-HA quickly accumulated in the liver (Fig. 1A), while 60 minutes after the administration, approximately all of radioactivity was excreted from the liver, and existed in the kidney and the bladder (Fig. 1B). On the other hand, in the case of [^{99m}Tc]MAG-HA, there were confirmed that, just after the administration, radoactivity did not accumulated in the liver, but was excreted from the kidney into bladder (Fig. 1C, 1D). Similarly, in the case of administered [^{99m}Tc]MAMA-BA in which the side-chain is shorter than 2 carbon atoms as compared with [^{99m}Tc]MAMA-HA, the former compound of [^{99m}Tc]MAMA-BA was hardly taken up by the liver, thus the kidney and bladder were visualized in an early stage after the administration (Fig. 1E). Further, 60 minutes after the administration, only the bladder was visualized (Fig. 1F).

### Example 11

### Determination of biodistribution

[^{99m}Tc]MAMA-HA (37 kBq/0.1 mL of physiological saline) was administered to the caudal vein of rat, then decapitated after a lapse of predetermined time. The blood and the organs of interest were excised. After measured the weight of these samples, the radioactivites were determined. After decay correction of the radioactivity, the results were evaluated by calculating %ID/g. The results of biodistribution of [^{99m}Tc]MAMA-HA in normal rats are shown in Table 2. After the administration, [^{99m}Tc]MAMA-HA shown higher uptake by the liver (10.59±0.02% ID/g, 5 minutes after the administration), and was eliminated rapidly from the liver with a lapse of time. Further, [^{99m}Tc]MAMA-HA was rapidly eliminated from the blood and high radioactivity was observed in the kidney. While in other organs, including the intestine, remarkable accumulation of the radioactivities were not observed.

**Table 2**

| Biodistribution of [^{99m}Tc]MAMA-HA in normal rats | | | |
|---|---|---|---|
| Tissues | 5 Minutes | 20 Minutes | 60 Minutes |
| Blood | 0.30±0.02 | 0.21±0.04 | 0.16±0.09 |
| Liver | 10.59±0.74 | 4.30±1.13 | 2.16±0.88 |
| Kidney | 8.16±1.42 | 5.80±0.86 | 5.25±2.60 |
| Spleen | 0.06±0.01 | 0.06±0.03 | 0.05±0.05 |
| Pancreas | 0.13±0.08 | 0.08±0.02 | 0.09±0.08 |
| Lunges | 0.15±0.01 | 0.14±0.07 | 0.12±0.06 |
| Heart | 0.15±0.02 | 0.11±0.02 | 0.07±0.04 |
| Intestine | 0.17±0.01 | 0.50±0.19 | 1.38±0.01 |
| Note: Measured value shows "Mean value ± SD of % dose/g" for 3 rats (percentage of the injected dose of radioactivity divided by tissue weight). | | | |

### Example 12

### Determination of the metabolites by in vivo test

[^{99m}Tc]MAMA-HA (15 MBq/0.5 mL of physiological saline) was administered to the caudal vein of rat, 60 minutes after, the rat was celitomized and urine was sampled from the urinary bladder. The urine sample was filtrated to remove proteins through a ultrafilter (Molcut II LGC: manufactured by Nihon Millipore Co.) having ability for cutting off micro-materials having larger than 10 kDa in size, and analyzed by reversed phase HPLC under the same conditions as in labeling operation.

Typical HPLC chromatograms of [^{99m}Tc]MAMA-HA, [^{99m}Tc]MAMA-BA, and [^{99m}Tc]MAMA-AA are shown in Fig. 2A. The radioactive metabolites in the urine sample obtained from the urinary bladder at 60 Minutes after the administration of [^{99m}Tc]MAMA-HA were observed at the retention times of 17 minutes and 23 minutes with the reversed phase HPLC analysis. Thus, 82.9 ± 9.0% of radioactivity in the urine exists in the fraction of retention time of 17 minutes (Fig. 2B).

Further, the urine sample and the authentic sample of [^{99m}Tc]MAMA-BA were analyzed at the same time by the reversed phase HPLC, the obtained HPLC chromatograms showed that 2 peaks of the radioactivity at the retention time of 17 minutes and 23 minutes. While from the results of simultaneous analysis of the urine sample and [^{99m}Tc]MAMA-AA, the third peak at the retention time of 16 minutes was observed in addition to the 2 peakes observed above (Fig. 2C and Fig. 2D).

### Example 13

### Determination of the metabolites by in vitro test

Under anesthetized, a rat was subjected to celiotomy and bleeded by perfusion via the portal vein with 20 mM of tris-HCl buffer solution (containing 100 mM of potassium chloride, 5 mM of magnesium chloride, 5 mM of potassium dihydrogen phosphate, pH 7.4) which is saturated with O₂/CO₂ (95/5) gas. Then, the liver was excised and pieces of liver slice having the thickness of 300 µm were prepared by using Stadie, Riggs type of slicer (manufactures by NATSUME SEISAKUSHO). After weighing, each pieces of liver slices were divided into two groups. One is inhibiting group which was preincubated in 20mM tris-HCl buffer solution at 37°C for 5 minutes, in the presence of 2-bromooctanoic acid (the final concentration: 100 µM) as β-oxidation inhibitor, and another one is control group which was preincubated in 20 mM tris-HCl buffer solution at 37°C for 5 minutes, without the inhibitor. Next, [^{99m}Tc]MAMA-HA (3.7 MBq) was added, and incubation was carried out under bubbling O₂/CO₂ (95/5) gas at 37°C for 60 minutes with shaking 60 times per minute. After addition of 5 mL of acetonitrile, the piece of liver slice was homogenized and centrifuged at 1000 × g for 20 minutes to precipitate proteins. The supernatent was concentrated under reduced pressure, then filtered by use of a filter having pore size of 0.22 µm (Hospital/ Pharmacy Milles GV, manufactured by Millipore Co.), so that samples for measurement were prepared. The samples were analyzed by reversed phase HPLC under the same conditions employed in Determination of metabolites by in vivo test. Survival rate of the cells in the liver slice sample used in the test was determined by measuring the activity of lactic acid dehydrogenase (LDH) leaked into the culture medium. At the termination point of experiments, it was not observed at the significant differences in LDH activities between the inhibition group and control group. (Inhibition group: 3,285 ± 367 IU/L, Control group: 3,175 ± 478 IU/L).

In conducting the in vitro experiment for determining the metabolite more than 90% of the radioactivity in liver homogenate was extracted.

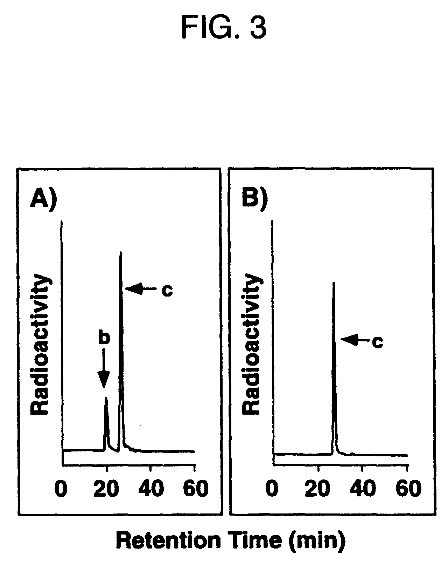

Reversed phase HPLC analysis of the sample from the control group showed two radioactive peak at the retention time of 17 minutes and 23 minutes (Fig. 3-A). 21.2 ± 3.0% of the whole radioactivity was eluted at the fraction retained at 17 minutes. On the other hand, the fraction retained at 17 minutes was not observed in the sample of inhibition group, and only the radioactive peak due to unchanged [^{99m}Tc]MAMA-HA was observed (Fig. 3-B).

## Claims

1. A fatty acid derivative labelled with a radioactive transition metal which is recognized as an energy substrate in a living body and is metabolized by β-oxidation, such that any change in said β-oxidation activity can be detected as a change in the elimination rate of the radioactivity from the body.

2. A derivative according to claim 1 which is of formula (1),
Q-RCOOH (1)
wherein:
Q is a chelating group capable of forming a neutral complex with a radioactive transition metal;
R is of formula -(CR¹R²)ₙ- wherein n is an integer of 4-9; and
R¹ and R² are each individually a hydrogen atom or a straight or branched chain lower alkyl group.

3. A derivative according to claim 2 wherein the lower alkyl group is a C₁-C₆ alkyl group.

4. A derivative according to claim 2 or 3 wherein the lower alkyl group is methyl, ethyl, n-propyl, i-propyl, n-butyl, s-butyl, t-butyl, pentyl or hexyl.

5. A derivative according to any one of claims 2 to 4 wherein the chelating group Q is selected from bisaminoethanethiol, monoamino-monoamide-bisthiol, and bisamido-bisthiol.

6. A derivative according to any one of claims 1 to 5 wherein the radioactive transition metal is technetium or rhenium.

7. A derivative according to any one of claims 2 to 6 wherein the chelating group Q is N-[[[2-[(triphenylmethyl)thio]ethyl]amino]acetyl]-S-(triphenylmethyl)-2-aminoethanethiol.

8. A diagnostic agent for radioisotope imaging which comprises a derivative as claimed in any one of claims 1 to 7 and a physiologically acceptable carrier or diluent.

9. A process for producing a fatty acid derivative of formula (1),
Q-RCOOH (1)
wherein:
Q is a chelating group capable of forming a neutral complex with a radioactive transition metal;
R is a group of the formula -(CR¹R²)ₙ- wherein, n is an integer of 4-9; and
R¹ and R² are each individually a hydrogen atom or a straight-chain or branched chain lower alkyl group;
which process comprises treating cysteamine with a halogenated acetyl halide to produce a monoaminemonoamide compound, and treating said monoaminemonoamide compound with a ω-halogenated fatty acid ester.

10. A process according to claim 9 wherein the halogen is bromine.
